# FASCICULE DE BREVET EUROPEEN

(11) **EP 0 544 587 B1**
(45) Date de publication et mention de la délivrance du brevet: **29.05.1996**
(21) Numéro de dépôt: 92403165.1
(22) Date de dépôt: 25.11.1992
(51) Int. Cl.: C07D 277/30, C07C 69/734, C07D 271/06, C07F 9/6539, C07F 9/40, C07C 69/738, A01N 43/78, A01N 43/82, A01N 37/10

(54) **Dérivés de l'acide alpha-méthylène-2-vinyl-benzèneacétique, leur procédé de préparation et leur application comme pesticides**
Alpha-Methylen-2-Vinyl-Phenylessigsäurederivate, Verfahren zu deren Herstellung und ihre Anwendung als Pestizide
Alpha-methylene-2-vinyl-benzeneacetic acid derivatives, process for their preparation and their use as pesticides

(30) Priorité: 26.11.1991 FR 9114556
(43) Date de publication de la demande: 02.06.1993
(73) Titulaire: ROUSSEL UCLAF, F-93230 Romainville (FR)
(72) Inventeur: Brayer, Jean-Louis, F-60440 Nanteuil le Haudouin (FR); Demoute, Jean-Pierre, F-93360 Neuilly Plaisance (FR); Le Stanc, Yannick, F-77340 Pontault Combault (FR); Reinier, Nicole, Résidence Val Fleuri, F-13009 Marseille (FR)
(74) Mandataire: Vieillefosse, Jean-Claude

(56) Documents cités:
- EP-A- 0 019 090
- EP-A- 0 178 826
- EP-A- 0 251 082
- EP-A- 0 278 595
- EP-A- 0 402 246
- JOURNAL OF THE CHEMICAL SOCIETY, PERKIN TRANSACTIONS 1 no. 6, Juin 1986, LETCHWORTH GB pages 913 - 917 G.MICHAEL BLACKBURN ET AL 'Synthesis of alpha- and gamma-fluoroalkylphosphonates'

## Description

La présente invention concerne de nouveaux dérivés de l'acide alpha-méthylène-2-vinyl-benzèneacétique, leur procédé de préparation et leur application comme pesticides.

On connaissait déjà des dérivés de l'acide alpha-méthylène benzèneacétique doués de propriétés fongicides (cf EP 178826).

L'invention a pour objet les composés de formule (I) : 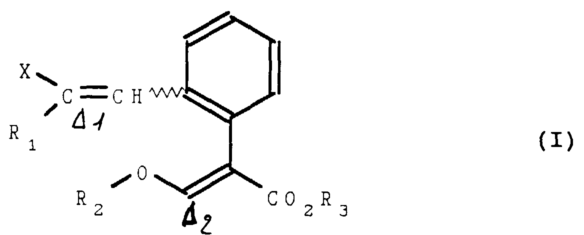 dans laquelle :
- X représente un atome d'halogène,
- R₂ et R₃ identiques ou différents l'un de l'autre représentent un radical alkyle linéaire, ramifié ou cyclique renfermant jusqu'à 8 atomes de carbone éventuellement substitué par un ou plusieurs atomes d'halogène, et
- ou R₁ représente un radical alkyle, alkényle ou alkynyle linéaire, ramifié ou cyclique, renfermant jusqu'à 12 atomes de carbone, éventuellement substitué par un ou plusieurs atomes d'halogène,
- ou R₁ représente un radical aryle ou hétéroaryle à 5 ou 6 chaînons, éventuellement substitués par un ou plusieurs substituants choisis dans le groupe constitué par les atomes d'halogène, par les radicaux alkyle, alkényle ou alkynyle linéaires, ramifiés ou cycliques ayant jusqu'à 8 atomes de carbone, eux-mêmes éventuellement substitués par un ou plusieurs atomes d'halogène, par les radicaux NO₂, NH₂, CN, OR', SR", SOR", SO₂R" et NR"'₂ (R', R" et R"' représentant des radicaux alkyle, alkényle ou alkynyle linéaires, ramifiés ou cycliques, renfermant jusqu'à 12 atomes de carbone,
éventuellement substitués par un ou plusieurs atomes d'halogène) et par les radicaux Ar, OAr₁ et SAr₂, dans lesquels les radicaux Ar, Ar₁ et Ar₂ représentent des radicaux aryles ou hétéroaryles renfermant jusqu'à 14 atomes de carbone éventuellement substitués par un ou plusieurs substituants choisis dans le groupe constitué par les atomes d'halogène, par les radicaux alkyle, alkényle ou alkynyle linéaires, ramifiés ou cycliques ayant jusqu'à 8 atomes de carbone, eux-mêmes éventuellement substitués par un ou plusieurs atomes d'halogène, par les radicaux NO₂, NH₂, CN, OR', SR", SOR", SO₂R" et NR"'₂ (R', R" et R"' représentant des radicaux alkyle, alkényle ou alkynyle linéaires, ramifies ou cycliques, renfermant jusqu'à 12 atomes de carbone, éventuellement substitués par un ou plusieurs atomes d'halogène) sous toutes les formes stéréoisomères possibles ainsi que leurs mélanges.

La géométrie des doubles liaisons Δ₁ et Δ₂ est E ou Z ou un mélange de géométrie E et Z.

L'atome d'halogène est de préférence un atome de fluor, de chlore ou de brome.

Dans la définition des différents substituants :
- alkyle représente de préférence un radical méthyle, éthyle, propyle, isopropyle, butyle, isobutyle, sec-butyle ou tert-butyle,
- alkényle représente de préférence un radical vinyle, allyle ou 1,1-diméthylallyle,
- alkynyle représente de préférence un radical éthynyle ou propynyle,
- aryle représente de préférence un radical phényle ou naphtyle,
- hétéroaryle représente de préférence un radical pyridyle, pyrimidyle, pyridazinyle, oxazolyle, thiazolyle, isoxazolyle, isothiazolyle, (1,3,4)oxadiazolyle, (1,2,4)oxadiazolyle, thiadiazolyle ou triazolyle,
- alkyle substitué par un ou plusieurs atomes d'halogène représente de préférence le radical CF₃.

L'invention a plus particulièrement pour objet les composés de formule (I) dans lesquels la double liaison Δ₂ est de géométrie E, les composés de formule (I) dans lesquels R₂ représente un radical méthyle, les composés de formule (I) dans lesquels R₃ représente un radical méthyle, les composés de formule (I) dans lesquels X représente un atome de fluor, les composés de formule (I) dans lesquels R₁ représente un radical phényle éventuellement substitué par un ou plusieurs radicaux choisis dans le groupe constitué par les atomes d'halogène et les radicaux CF₃, les composés de formule (I) dans lesquels R₁ représente un radical thiazolyle éventuellement substitué par un ou plusieurs des radicaux indiqués précédemment, par exemple un radical thiazolyle substitué par un ou plusieurs radicaux choisis dans le groupe constitué par les atomes d'halogène et le radical phényle éventuellement substitué par un ou plusieurs radicaux choisis dans le groupe constitué par les atomes d'halogène, les radicaux CF₃ et OCF₃, les composés de formule (I) dans laquelle R₁ représente un radical oxadiazolyle éventuellement substitué par un ou plusieurs des radicaux indiqués précédemment, par exemple un radical oxadiazolyle substitué par un radical phényle éventuellement par un ou plusieurs radicaux choisis dans le groupe constitué par les atomes d'halogène et les radicaux CF₃ et OCF₃.

L'invention a particulièrement pour objet les composés de formule (I) dont la préparation est donnée ci-après dans la partie expérimentale et plus particulièrement les produits des exemples 1, 4, 5, 7, 18, 20, 25, 26 et 30.

L'invention a également pour objet un procédé de préparation caractérisé en ce que l'on soumet un composé de formule (II) : 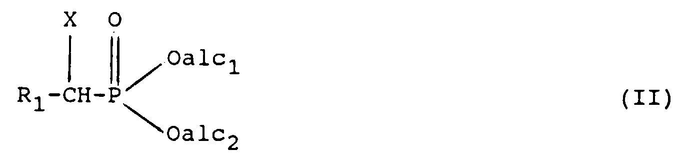 dans laquelle R₁ et X conservent leur signification précédente et alc₁ et alc₂ représentent un radical alkyle renfermant jusqu'à 8 atomes de carbone, à l'action d'un composé de formule (III) : 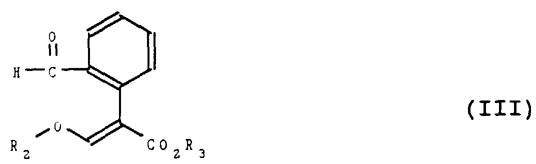 dans laquelle R₂ et R₃ conservent leur signification précédente, en présence d'un agent basique, pour obtenir le composé de formule (I) correspondant, que l'on sépare si désiré en chacun de ses isomères et le cas échéant transforme l'un des isomères en l'autre isomère.

Dans un mode de réalisation préféré du procédé de l'invention, l'agent basique est une base forte telle que l'hydrure de sodium, un alcoolate alcalin ou alcalinoterreux comme le terbutylate de potassium ou le terbutylate de sodium, un amidure alcalin ou un dérivé organolithien.

Les produits de formule (I) dans lesquels R₁ représente un radical aryle ou hétéroaryle monosubstitué peuvent être si désiré soumis à l'action d'un réactif capable d'introduire un autre substituant. Il peut s'agir par exemple d'une halogénation telle qu'une bromation effectuée au sein d'un solvant organique, par exemple le tétrachlorure de carbone.

Les produits de formule (I) dans lesquels R₁ représente un radical aryle ou hétéroaryle substitué par un atome d'halogène peuvent être si désiré, soumis à une réaction d'alkénylation, d'alkynylation, d'arylation ou d'hétéroarylation en effectuant une réaction de couplage organométallique catalysée ou palladium.

La séparation des isomères est effectuée selon les méthodes classiques connues de l'homme du métier.

L'isomérisation des doubles liaisons fluorées est réalisée au moyen d'une quantité catalytique de brome en présence de radiations lumineuses.

Les composés de formule (II) sont des produits connus d'une façon générale.

Des exemples détaillés de préparation de composés de formule (II) sont indiqués ci-après dans la partie expérimentale. Les produits de formule (II) peuvent être préparés selon les schémas réactionnels suivants :

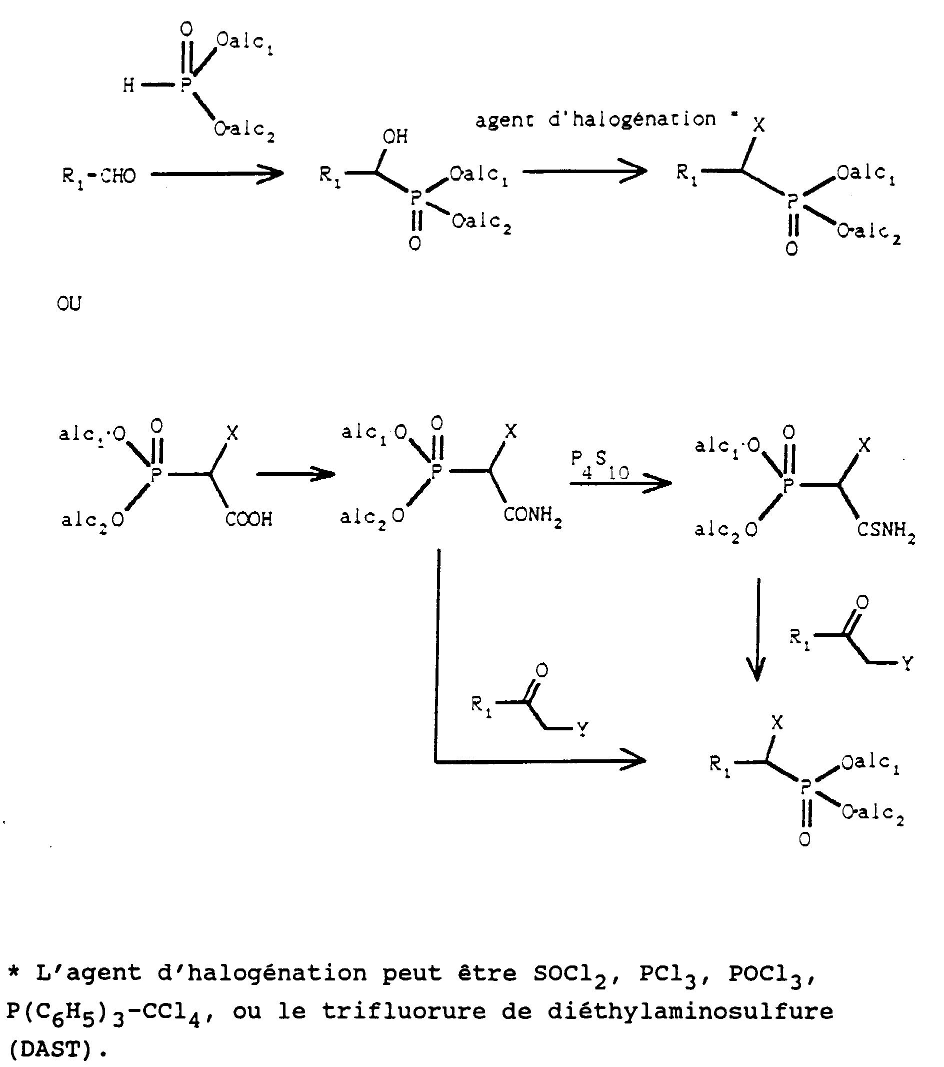

Les composés de formule (III) sont des produits connus d'une façon générale. Des exemples détaillés de préparation de composés de formule (III) sont indiqués ci-après dans la partie expérimentale. Les produits de formule (III) peuvent être préparés selon les schémas réactionnels suivants :

### 1ère METHODE

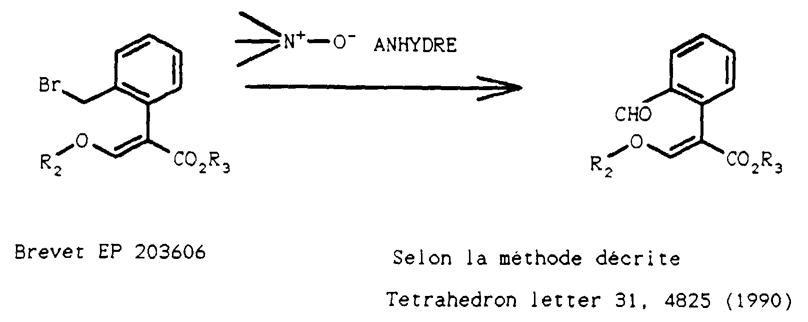

### 2ème METHODE

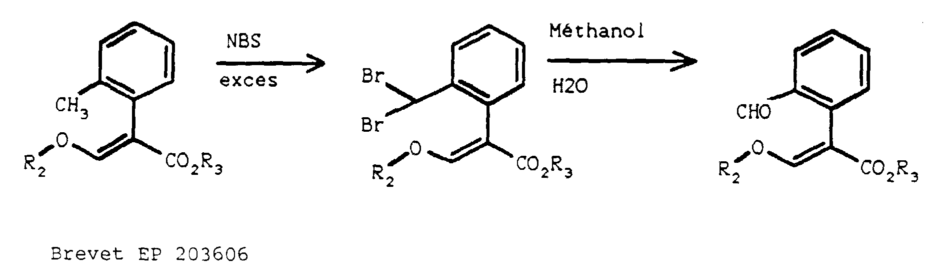

Les composés de formule (I) présentent d'intéressantes propriétés pesticides notamment insecticides, acaricides et nématicides. L'invention a donc pour objet les compositions pesticides renfermant comme principe actif au moins un composé de formule (I) défini précédemment et notamment les compositions pesticides renfermant comme principe actif au moins un composé de l'exemple 1, 4, 5, 7, 18, 20, 25, 26 ou 30.

Les produits de formule (I) peuvent donc être utilisés notamment pour lutter contre les insectes dans le domaine agricole, pour lutter par exemple contre les pucerons, les larves de lépidoptères et les coléoptères. Ils sont utilisés à des doses comprises entre 10 g et 1000 g de matière active à l'hectare.

Les produits de formule (I) peuvent également être utilisés pour lutter contre les insectes dans les locaux, pour lutter notamment contre les mouches, les moustiques et les blattes.

Les produits de formule (I) peuvent aussi être utilisés pour lutter contre les acariens parasites des végétaux.

Les composés de formule (I) peuvent aussi être utilisés pour lutter contre les nématodes parasites des végétaux.

Les composés de formule (I) peuvent encore être utilisés pour lutter contre les acariens parasites des animaux, pour lutter par exemple contre les tiques et notamment les tiques de l'espèce Boophilus, celles de l'espèce Hyalomnia, celles de l'espèce Amblyomnia et celles de l'espèce Rhipicephalus, ou pour lutter contre toutes sortes de gales et notamment la gale sarcoptique, la gale psoroptique et la gale chorioptique.

L'invention a donc également pour objet les compositions destinées à la lutte contre les parasites des animaux à sang chaud, les parasites des locaux et des végétaux, caractérisées en ce qu'elles renferment au moins l'un des produits définis ci-dessus.

L'invention a notamment pour objet les compositions insecticides renfermant comme principe actif au moins l'un des produits définis ci-dessus.

Les compositions selon l'invention sont préparées selon les procédés usuels de l'industrie agrochimique ou de l'industrie vétérinaire ou de l'industrie des produits destinées à la nutrition animale.

Ces compositions peuvent se présenter sous forme de poudres, granulés, suspensions, émulsions, solutions, solutions pour aérosols, bandes combustibles, appâts ou autres préparations employés classiquement pour l'utilisation de ce genre de composés.

Outre le principe actif, ces compositions contiennent, en général, un véhicule et/ou un agent tensio-actif, non ionique, assurant, en outre, une dispersion uniforme des substances constitutives du mélange. Le véhicule utilisé peut-être un liquide, tel que l'eau, l'alcool, les hydrocarbures ou autres solvants organiques, une huile minérale, animale ou végétale, une poudre telle que le talc, les argiles, les silicates, le kieselguhr ou un solide combustible.

Les compositions insecticides selon l'invention contiennent de préférence de 0,005 % à 10 % en poids de matière active.

Selon un mode opératoire avantageux, pour un usage dans les locaux, les compositions selon l'invention sont utilisées sous forme de compositions fumigantes.

Les compositions selon l'invention peuvent alors être avantageusement constituées, pour la partie non active, d'un serpentin insecticide (ou coil) combustible, ou encore d'un substrat fibreux incombustible. Dans ce dernier cas, le fumigant obtenu après incorporation de la matière active est placé sur un appareil chauffant tel qu'un électromosquito destroyer.

Dans le cas où l'on utilise un serpentin insecticide, le support inerte peut être, par exemple, composé de marc de pyrèthre, poudre de Tabu (ou poudre de feuilles de Machilus Thumbergii), poudre de tige de pyrèthre, poudre de feuille de cèdre, poudre de bois (telle que la sciure de pin) amidon et poudre de coque de noix de coco. La dose de matière active peut alors être, par exemple, de 0,03 à 1 % en poids.

Dans le cas où l'on utilise un support fibreux incombustible, la dose de matière active peut alors être, par exemple, de 0,03 à 95 % en poids.

Les compositions selon l'invention pour un usage dans les locaux peuvent aussi être obtenues en préparant une huile pulvérisable à base de principe actif, cette huile imbibant la mèche d'une lampe et étant alors soumise à la combustion.

La concentration du principe actif incorporé à l'huile est, de préférence, de 0,03 à 95 % en poids.

L'invention a également pour objet, les compositions acaricides renfermant comme principe actif au moins un des produits de formule (I) définie ci-dessus.

L'invention a également pour objet les compositions nématicides renfermant comme principe actif au moins un des produits de formule (I) ci-dessus.

Les compositions insecticides selon l'invention, comme les compositions acaricides et nématicides peuvent être additionnées éventuellement d'un ou plusieurs autres agents pesticides. Les compositions acaricides et nématicides peuvent se présenter notamment sous forme de poudre, granulés, suspensions, émulsions, solutions.

Pour l'usage acaricide, on utilise de préférence des poudres mouillables, pour pulvérisation foliaire, contenant de 1 à 80 % ou des liquides pour pulvérisation foliaire contenant de 1 à 500 g/l de principe actif. On peut également employer des poudres pour poudrage foliaires contenant de 0,05 à 3 % de matière active.

Pour l'usage nématicide, on utilise de préférence des liquides pour traitement des sols contenant de 300 à 500 g/l de principe actif.

Les composés acaricides et nématicides selon l'invention sont utilisés, de préférence, à des doses comprises entre 1 et 100 g de matière active à l'hectare.

L'invention a également pour objet les compositions acaricides destinées à la lutte contre les acariens parasites des animaux à sang chaud, notamment contre les tiques et les gales, caractérisées en ce qu'elles renferment comme principe actif, au moins l'un des produits de formule (I) définie ci-dessus.

Les exemples suivants illustrent l'invention sans toutefois la limiter.

### EXEMPLE 1 : 2-[2-[4-bromo 3-(trifluorométhyl) phényl] 2-fluoro éthényl] alpha-(méthoxyméthylène) benzène acétate de méthyle isomère Z,E et isomère E,E

On introduit à -60°C une solution renfermant 1,3 g de terbutylate de potassium et 10 ml de tétrahydrofuranne dans une solution renfermant 4,3 g de [[4-bromo 3-(trifluorométhyl) phényl] fluorométhyl] phosphonate de diéthyle dont la préparation est donnée ci-après (préparation 2) et 20 ml de tétrahydrofuranne. On maintient le mélange réactionnel sous agitation pendant 15 minutes à -60°C et introduit sans dépasser -50°C, une solution renfermant 2,4 g de 2-formyl alpha-(méthoxyméthylène) benzène acétate de méthyle (préparation 1) et 15 ml de tétrahydrofuranne. On laisse la température du mélange réactionnel remonter à 20 ≃ 25°C et agite à 20-25° pendant 1 heure et verse sur un mélange eau + acide chlorhydrique 1N (100 ml) et éther isopropylique 200 ml. On décante et extrait à l'éther isopropylique. On sèche et chromatographie sur silice en éluant avec le mélange hexane-acétate d'éthyle (8-2). On obtient ainsi les produits recherchés. F = 115,5°C isomère Z,E, F = 103,1°C isomère E,E.

### EXEMPLE 2: 2-[2-[2-(3,4-dichlorophényl) 4-thiazolyl] 2-fluoro éthényl] alpha-(méthoxyméthylène) benzène acétate de méthyle isomère Z,E et isomère E,E

On introduit à 0°C, une solution renfermant 0,7 g de terbutylate de potassium et 6 ml de tétrahydrofuranne dans un mélange renfermant 1,33 g du produit de la préparation 1, 2,4 g du produit de la préparation 3 (ou 2-(3,4-dichlorophényl 4-thiazolyl) fluorométhyl phosphonate de diéthyle) et 10 ml de tétrahydrofuranne. On maintient le mélange réactionnel pendant 1 heure à 0 + 5°C et le verse sur un mélange 100 ml d'acide chlorhydrique 1N et 100 ml de chlorure de méthylène. On décante et extrait la phase aqueuse avec 2 fois 100 ml de chlorure de méthylène. On sèche et amène à sec. On chromatographie sur silice en éluant avec le mélange hexane-éther isopropylique (7-3). On obtient ainsi 0,6 g d'isomère Z,E F = 153,2°C, et 0,6 g d'isomère E,E F = 112,1°C.

### EXEMPLE 3: 2-[2-[2-(2,4-dichlorophényl) 4-thiazolyl] 2-fluoro éthényl] alpha-(méthoxyméthylène) benzène acétate de méthyle isomère Z,E et mélange Z,E et E,E

On introduit à 0 + 5°C, une solution renfermant 0,57 g de terbutylate de potassium et 5 ml de tétrahydrofuranne dans une solution renfermant 1,06 g du produit de la préparation 1 et 1,9 g de 2-(2,4-dichlorophényl 4-thiazolyl] fluorométhyl phosphonate de diéthyle obtenu comme à la préparation 3 à partir de 2-[2-(2,4-dichlorophényl) 4-formyle thiazole et 10 ml de tétrahydrofuranne. On maintient le mélange réactionnel sous agitation pendant 3 heures à 0 + 5°C. On verse le mélange réactionnel sur un mélange 1N d'acide chlorhydrique et 100 ml d'éther isopropylique. On décante et extrait à l'éther isopropylique. On sèche sur sulfate de sodium et amène à sec. On chromatographie sur silice en éluant avec le mélange hexane-éther isopropylique (7-3). On obtient ainsi 0,9 g de produit que l'on chromatographie sur silice en éluant avec le mélange hexane-éther isopropylique (8-2). On obtient ainsi 0,11 g de produit recherché Z,E et 0,57 g de mélange 75-25 E,E et Z,E F = 123,8°C.

### EXEMPLE 4 : 2-[2-fluoro 2-[4-thiazolyl 2-(4-trifluoro méthoxy) phényl] éthényl] alpha-(méthoxyméthylène) benzène acétate de méthyle, isomère Z,E et isomère E,E

On ajoute à 0°C une solution renfermant 0,53 g de tertiobutylate de potassium et 2,4 ml de tétrahydrofuranne dans un mélange renfermant 0,92 mg de 2-formyl alpha-(méthoxyméthylène) benzène acétate de méthyle (préparation 1), 1,8 g de [fluoro [4-[4-trifluorométhoxy phényl] 2-thiazolyl] méthyl] phosphonate de diéthyle (préparation 4) et 3 ml de tétrahydrofuranne. On maintient le mélange réactionnel sous agitation pendant 16 heures. On acidifie à pH 4 par addition d'acide chlorhydrique 1N. On extrait au chlorure de méthylène. On lave à l'eau et sèche. On élimine le solvant et obtient 2,3 g de produit que l'on chromatographie sur silice en éluant avec le mélange hexane-acétate d'éthyle (85-15). On obtient ainsi 0,2 g de produit Z,E, F = 137,9°C et 0,44 g de produit E,E, F = 99,5°C.

### EXEMPLE 5 : 2-[2-fluoro 2-(4-phényl 2-thiazolyl) éthényl] alpha-(méthoxyméthylène) benzène acétate de méthyle, mélange (Z+E),E

On ajoute 233 mg d'hydrure de sodium à 50 % dans l'huile dans une solution renfermant 1,07 g de 2-formyl alpha-(méthoxyméthylène) benzène acétate de méthyle (préparation 1), 1,6 g de fluoro [4-phényl 2-thiazolyl] méthyl] phosphonate de diéthyle (préparation 5) et 10 ml de tétrahydrofuranne. On maintient le mélange réactionnel sous agitation pendant 16 heures. On le verse dans une solution aqueuse d'acide chlorhydrique. On extrait au chlorure de méthylène. On sèche. On élimine le solvant. On obtient 2,27 g de produit que l'on chromatographie sur silice en éluant avec le mélange hexane-acétate d'éthyle (80-20). On obtient 1,46 g d'une huile que l'on traite au pentane. On obtient 1,07 g de produit recherché fondant à 94°C.

### EXEMPLE 6 : alpha-(méthoxyméthylène) 2-[2-fluoro 2-[4-(1-méthyléthyl) 2-thiazolyl] éthényl] benzène acétate de méthyle, isomère Z,E et isomère E,E

On ajoute goutte à goutte une solution renfermant 1,45 g de tertiobutylate de potassium et 6 ml de tétrahydrofuranne dans un mélange renfermant 2,83 g de 2-formyl alpha-(méthoxyméthylène) benzène acétate de méthyle, 3,8 g de fluoro [4-(1-méthyl éthyl) 2-thiazolyl] méthyl] phosphonate de diéthyle (préparation 6) et 15 ml de tétrahydrofuranne. On laisse le mélange réactionnel sous agitation pendant une nuit. On amène à pH 4. On extrait au chlorure de méthylène. On lave et sèche. On élimine le solvant. On obtient 5,23 g de produit que l'on chromatographie sur silice en éluant avec le mélange hexaneacétate d'éthyle (85-15). On obtient 0,34 g d'isomère Z,E, F = 104,3°C et 1 g d'isomère E,E.

### EXEMPLE 7: 2-[2-[4-(3,4-dichlorophényl) 2-thiazolyl] 2-fluoro éthényl] alpha-(méthoxyméthylène) benzène acétate de méthyle isomère Z,E et isomère E,E

On ajoute 326 mg d'hydrure de sodium à 50 % dans l'huile dans une solution renfermant 1,5 g de 2-formyl alpha-(méthoxyméthylène) benzène acétate de méthyle (préparation 1), 2,7 g de [[4-(3,4-dichlorophényl) 2-thiazolyl] fluorométhyl] phosphonate de diéthyle (préparation 7) et 10 ml de tétrahydrofuranne. On maintient le mélange réactionnel sous agitation pendant une nuit. On verse dans l'eau. On extrait au chlorure de méthylène. On sèche les phases organiques. On élimine le solvant. On obtient ainsi 3,66 g de produit que l'on chromatographie sur silice en éluant avec le mélange hexane-acétate d'éthyle (85-15). On obtient ainsi 0,22 g d'isomère Z,E F = 192,3°C, 1,52 g d'isomère E,E, F = 115,6°C.

En opérant comme indiqué dans les exemples précédents, à partir 2-formyl alpha-(méthoxyméthylène) benzène acétate de méthyle et du phosphonate approprié, on a préparé l'un des produits suivants.

### EXEMPLE 8: 2-[2-fluoro 2-[2-(trifluorométhyl) 4-thiazolyl] éthényl] α-(méthoxyméthylène benzèneacétate de méthyle - isomère (Z,E) et isomère (E,E).

isomère Z,E : F = 143°C.

### EXEMPLE 9: 2-[2-[4-[[1,1'-biphényl]-4-yl] 2-thiazolyl] 2-fluoro éthényl] α-(méthoxyméthylène benzèneacétate de méthyle - isomère (Z,E) et isomère (E,E).

isomère Z,E : F = 121,6°C.

isomère E,E : F = 139,5°C.

### EXEMPLE 10: 2-[2-[4-(4-chlorophényl) 2-thiazolyl] 2-fluoroéthényl] α-(méthoxyméthylène) benzèneacétate de méthyle, isomère (Z,E) et isomère (E,E).

isomère Z,E : F = 137,2°C.

isomère E,E : F = 116,5°C.

### EXEMPLE 11: 2-[2-[4-(4-bromophényl) 2-thiazolyl] 2-fluoroéthényl] α-(méthoxyméthylène) benzèneacétate de méthyle, isomère (Z,E) et isomère (E,E).

isomère Z,E : F = 145,8°C.

isomère E,E : F = 132,5°C.

### EXEMPLE 12: 2-[2-fluoro 2-[4-(trifluorométhyl) 2-thiazolyl] éthényl] α-(méthoxyméthylène) benzèneacétate de méthyle, isomère (Z,E) et isomère (E,E).

isomère Z,E : F = 109°C.

### EXEMPLE 13: 2-[[2'-éthyl(2,4'-bithiazole)-4-yl] 2-fluoroéthényl] α-(méthoxyméthylène) benzèneacétate de méthyle, mélange d'isomères (Z,E).

mélange d'isomères Z,E (80-20) : F = 83,5°C.

### EXEMPLE 14: 2-[2-fluoro 2-[2-pyridinyl 4-thiazolyl] éthényl] α-(méthoxyméthylène) benzèneacétate de méthyle, isomère (Z,E) et isomère (E,E).

isomère Z,E : F = 146°C.

isomère E,E : F = 101°C.

### EXEMPLE 15: 2-[2-fluoro 2-[3-[4-(trifluorométhoxy) phényl] 1,2,4-oxadiazol-5-yl]] éthényl] α-(méthoxyméthylène) benzèneacétate de méthyle, isomère (E,E).

isomère E,E : F = 103,6°C.

### EXEMPLE 16: 2-[2-fluoro 2-[3-(trifluorométhyl) phényl] éthényl] α-(méthoxyméthylène) benzèneacétate de méthyle, isomère (Z,E) et isomère (E,E).

isomère Z,E : F = 90°C.

isomère E,E : F = 61,5°C.

### EXEMPLE 17: 2-[2-fluoro 2-[4-(4-nitrophényl] 2-thiazolyl] éthényl] α-(méthoxyméthylène) benzèneacétate de méthyle, mélange d'isomères (Z,E), E).

F = 79°C.

### EXEMPLE 18: 2-[2-[4-(4-fluorophényl) 2-thiazolyl] 2-fluoroéthényl]

### α-(méthoxyméthylène) benzèneacétate de méthyle, isomère (Z,E) et isomère (E,E).

isomère Z,E : F = 135,8°C.

isomère E,E : F = 99,8°C.

### EXEMPLE 19: α-(méthoxyméthylène) 2-[2-(3-bromophényl) 2-fluoroéthényl benzèneacétate de méthyle, isomère (Z,E) et isomère (E,E).

isomère Z,E : F = 129°C.

isomère E,E : F = 94°C.

### EXEMPLE 20: 2-[2-[5-bromo 4-(3,4-dichlorophényl) 2-thiazolyl] 2-fluoroéthényl] α-(méthoxyméthylène) benzèneacétate de méthyle, isomère (Z,E).

On mélange sous atmosphère inerte 930 mg de produit obtenu à l'exemple 7 dans 5 cm³ de tétrachlorure de carbone, ajoute 480 mg de brome en solution dans 2 cm³ de tétrachlorure de carbone et chauffe 4 heures à 60°C. On verse dans 50 cm³ d'eau, extrait au chlorure de méthylène, sèche et évapore le solvant. On chromatographie le résidu sur silice (éluant : hexane-éther isopropylique 7-3) et recueille 330 mg de produit attendu. F = 154,2°C.

### EXEMPLE 21: 2-[2-(3-éthynylphényl) 2-fluoroéthényl] α-(méthoxyméthylène) benzèneacétate de méthyle, isomère (Z,E).

### Stade A : 2-[2-fluoro 2-[3-[2-(triméthylsilyléthynyl) phényl] éthényl] α-(méthoxyméthylène) benzèneacétate de méthyle, isomère (Z,E).

On ajoute En 4 à 5 minutes, 2,3 cm³ de triméthylsilylacétylène à un mélange comprenant 1,96 g de produit préparé à l'exemple 19, 14 cm³ d'acétonitrile, 20 cm³ de triéthylamine, 0,54 g de palladium à 10% sur charbon actif, 48 mg d'iodure de cuivre, 0,22 g de triphénylphosphine. On chauffe 20 heures à 80°C dans une enceinte close, refroidit à 5°C, filtre, verse le filtrat dans l'eau, extrait au chlorure de méthylène, lave à l'eau, sèche et évapore le solvant. Après chromatographie sur silice (éluant : hexane-acétate d'éthyle 8-2), on obtient 0,95 g de produit attendu. F = 119°C.

### Stade B : 2-[2-(3-éthynylphényl) 2-fluoroéthényl] α-(méthoxyméthylène) benzèneacétate de méthyle, isomère (Z,E).

On introduit à -70°C une solution renfermant 1,6 cm³ de fluorure de terbutylammonium en solution 1M dans le tétrahydrofuranne, dans une solution renfermant 0,63 g de produit préparé comme indiqué au stade A et 20 cm³ de tétrahydrofuranne. On maintient le mélange réactionnel sous agitation pendant 3 heures à -70°C. On verse le mélange sur une solution aqueuse de dihydrogénophosphate de potassium refroidie à O°/+5°C, agite 15 minutes, extrait au chlorure de méthylène, lave à l'eau, sèche et évapore le solvant. Après chromatographie sur silice (éluant : hexane-acétate d'éthyle 8-2) et cristallisation dans du pentane, on obtient 0,35 g de produit attendu. F = 103°C.

### EXEMPLE 22: [4-(3,3-diméthyl 1-butynyl) 3-(trifluorométhyl) phényl] 2-fluoroéthényl α-(méthoxyméthylène) benzène acétate de méthyle, isomère (Z,E).

On chauffe 48 heures à 80°C dans une enceinte close, 0,92 g de produit (isomère Z,E) obtenu à l'exemple 1, 7 cm³ d'acétonitrile, 9 cm³ de triéthylamine, 0,2 g de charbon actif palladié, 0,1 g de triphénylphosphine, 0,02 g d'iodure de cuivre et 1 cm³ de terbutylacétylène. Après refroidissement, on filtre, évapore le solvant, chromatographie le résidu sur silice (éluant : chlorure de méthylène) et obtient 0,5 g de produit attendu, isomère (Z,E). F = 124°C.

### EXEMPLE 23: 2-[2-(3-éthénylphényl) 2-fluoroéthényl] α-(méthoxyméthylène) benzène acétate de méthyle, isomère (Z,E).

On chauffe au reflux à 110°C, 2,35 g de produit obtenu à l'exemple 19, 30 cm³ de toluène, 0,28 g de triphénylphosphine palladium et 2 cm³ de vinyltributylétain et agite 16 heures à cette température. On refroidit à 20°-25°C, évapore le solvant et obtient 5 g de produit brut que l'on chromatographie sur silice (éluant : éther isopropylique-hexane 65-35). On obtient 1,29 g de produit attendu. F = 86°C.

### EXEMPLE 24: 2-[2-[3-(3-trifluorométhylphényl) phényl] 2-fluoroéthényl] α-(méthoxyméthylène) benzène acétate de méthyle, isomère (Z,E).

### Stade A : 2-[2-(3-tributylstannanephényl) 2-fluoroéthényl] α-(méthoxyméthylène) benzène acétate de méthyle.

On opère comme à l'exemple 21 en utilisant au départ 5,9 g de produit obtenu à l'exemple 19, 90 cm³ de toluène, 9,8 cm³ d'hexabutyldistannane et 0,86 g de triphénylphosphine palladium et en chauffant pendant 48 heures à 110°C. Après chromatographie sur silice (éluant : chlorure de méthylène-hexane 6-4), on obtient 9 g de produit attendu.

### Stade B : 2-[2-[3-(3-trifluorométhylphényl) phényl] 2-fluoroéthényl] α-(méthoxyméthylène) benzène acétate de méthyle, isomère (Z,E).

On chauffe à 110°C pendant 16 heures 3,5 g de produit obtenu au stade A, 35 cm³ de toluène, 1,3 cm³ de 3-(trifluorométhyl) iodobenzène et 0,2 g de chlorure de bis(triphénylphosphine) palladium. On refroidit à température ambiante, filtre, évapore le solvant, chromatographie le résidu sur silice (éluant : chlorure de méthylène-hexane 65-35) et recueille 1,4 g de produit attendu. F = 119,5°C.

### EXEMPLE 25: 2-[2-[5-bromo 4-(4-fluorophényl) 2-thiazolyl] 2-fluoroéthényl] α-(méthoxyméthylène) benzène acétate de méthyle, isomère (Z,E) et isomère (E,E)

On opère comme à l'exemple 1 à partir de 11,36 g de 2-formyl α-(méthoxyméthylène) benzène acétate de méthyle, 22 g de [[5-bromo 4-(4-fluorophényl) 2-thiazolyl] fluorométhyl phosphonate de diéthyle (préparation 19) et 6,5 g de terbutylate de potassium. On obtient 26,6 g de produit brut que l'on chromatographie sur silice (éluant : hexane-éther isopropylique 1-1). On obtient 920 mg d'isomère (Z,E) F = 189°C, et 20 mg d'isomère (E,E) F = 133°C.

### EXEMPLE 26: 2-[2-fluoro 2-[3-[4-(trifluorométhoxy) phényl] 1,2,4-oxadiazol-5-yl] éthényl] α-(méthoxyméthylène) benzène acétate de méthyle, isomère (Z,E).

465 mg de produit obtenu à l'exemple 15 (isomère E,E) en solutiuon dans 5 cm³ de tétrachlorure de carbone sont placés sous radiations lumineuses (250 Watts) puis on ajoute 0,1 cm³ d'une solution à 5% de brome dans le tétrachlorure de carbone. En fin de réaction, le solvant est évaporé. Après traitement du résidu au pentane, on obtient 400 mg de produit attendu, isomère Z,E. F =137°C.

### EXEMPLE 27: 2-[2-[3-(trifluorométhyl) phényl 2-chloroéthényl] α-(méthoxyméthylène) benzène acétate de méthyle, isomère (Z,E) et isomère (E,E).

On refroidit à -70°/-75°C une solution comprenant 2,48 g de [3-(trifluorométhyl) phényl] chlorométhyl] phosphonate de diéthyle (préparation 20), 1,65 g de 2-formyl α-(méthoxyméthylène) benzèneacétate de méthyle (préparation 1) dans 20 cm³ de tétrahydrofuranne puis ajoute en 45 minutes 0,95 g de terbutylate de potassium en solution sans 10 cm³ de tétrahydrofuranne. On agite 1 heure à -65°/-70°C, verse sur un mélange eau + acide chlorhydrique 2N refroidi à 0°/-5°C, agite 15 minutes à pH 3-4. On extrait au chlorure de méthyl-ène, lave à l'eau jusqu'à neutralité, sèche, évapore le solvant, chromatographie le résidu sur silice (éluant : hexane-acétate d'éthyle 85-15) et obtient 1,48 g d'isomère (E,E) et 0,92 g d'isomère (Z,E) après cristallisation et lavage au pentane.

### EXEMPLE 28: 2-[2-[3-(trifluorométhyl) phényl 2-bromoéthényl] α-(méthoxyméthylène) benzène acétate de méthyle, isomère (E,E).

On opère comme à l'exemple 27 en utilisant au départ 2,81 g de [3-(trifluorométhyl) phényl] 2-bromométhyl] phosphonate de diéthyle (préparation 21) et 1,65 g de 2-formyl α-(méthoxyméthylène) benzène acétate de méthyle (préparation 1). On obtient 1,35 g de produit attendu.

### EXEMPLE 29: 2-[2-[3-(trifluorométhyl) phényl 2-bromoéthényl] α-(méthoxyméthylène) benzène acétate de méthyle, isomère (Z,E).

On ajoute 0,23 cm³ d'une solution à 5% de brome dans du tétrachlorure de carbone à 1 g de produit obtenu à l'exemple 28 (isomère E,E) dans 15 cm³ de tétrabromure de carbone. On place le milieu réactionnel sous radiation lumineuse (300 Watts) pendant 2 heures. On récupère après évaporation du solvant 1,2 g de produit brut que l'on purifie par chromatographie sur silice (éluant : hexane-acétate d'éthyle 8-2). On obtient 0,42 g de produit attendu. F = 94°C.

En opérant comme dans les exemples précédents, on a également préparé le produit suivant.

### EXEMPLE 30: 2-[2-fluoro 2-[3-phényl 1,2,4-oxadiazol-5-yl] éthényl] α-(méthoxyméthylène) benzène acétate de méthyle, isomère (Z,E).

F = 121°C.

### PREPARATION 1 : 2-formyl alpha-(méthoxyméthylène) benzène acétate de méthyle

On introduit à 0°C, 50,7 g d'une solution de 2-bromoéthyl alpha-(méthoxyméthylène) benzène acétate de méthyle (préparé selon le procédé décrit dans EP 203606, 100 cm³ de chlorure de méthylène dans un mélange de 55 g de triméthylamine N-oxide anhydre, 350 cm³ de diméthylsulfoxide et 160 cm³ de chlorure de méthylène. On maintient le mélange réactionnel sous agitation pendant 4 heures à 20 ≃ 25°C. On verse sur un mélange d'eau et de glace. On décante, extrait au chlorure de méthylène, sèche, amène à sec. On chromatographie sur silice le produit obtenu (éluant hexane-acétate d'éthyle (8-2). On obtient 17,5 g de produit recherché F = 81°C.

### PREPARATION 2 : [[4-bromo 3-(trifluorométhyl) phényl] fluorométhyl phosphonate de diéthyle.

### Stade A : 4-bromo 3-(trifluorométhyl) benzaldéhyde

### a) Préparation du chlorure de 4-bromo 3-(trifluorométhyl) benzène diazonium

On introduit 60 g de 4-bromo 3-(trifluorométhyl) aniline dans 60 cm³ d'eau. On introduit ensuite 57 cm³ d'acide chlorhydrique concentré. On obtient une suspension que l'on laisse revenir à 20°C. On ajoute de la glace. On ajoute à 0° ± 5°C, une solution renfermant 17,5 g de nitrite de sodium et 25 cm³ d'eau. On maintient sous agitation à 0° ± 5°C, pendant 15 minutes. On ajoute une solution renfermant 25 g d'acétate de sodium hydraté et 35 cm³ d'eau.

On ajoute entre 10°C et 15°C à cette solution, la solution aqueuse de formaldéhyde-oxime à 10 %, préparée de la manière suivante :

### b) Préparation d'une solution aqueuse de formaldéhyde-oxime à 10 %.

On introduit 26,3 g de chlorhydrate d'hydroxylamine dans 170 cm³ d'eau distillée. On ajoute 28,75 g de formaldéhyde à 40 %. On chauffe à 40°C. On ajoute 51 g d'acétate de sodium hydraté. On porte au reflux pendant 15 minutes. On laisse 16 heures au repos. On ajoute 6,5 g de sulfate de cuivre et 1 g de sulfate de sodium. On refroidit à 15°C et introduit une solution de 160 g d'acétate de sodium et 300 cm³ d'eau distillée.

On ajoute à 10/15°C, à la solution obtenue, en b) ci-dessus, la solution du sel de diazonium obtenue en a) ci-dessus, agite 1 heure à 15°C. On ajoute 230 cm³ d'acide chlrohydrique concentré et chauffe 2 heures au reflux. On refroidit et extrait avec 6 fois 400 cm³ d'éther éthylique. La solution organique est lavée avec une solution saturée de bicarbonate de sodium et à l'eau saturée de chlorure de sodium, séchée, filtrée et concentrée à sec. On obtient 66,3 g de produit que l'on chromatographie sur silice (éluant : hexane-acétate d'éthyle (95-5)). On obtient 11,15 g de produit recherché.

| | |
|---|---|
| Spectre IR CHCl₃ | |
| C=O | 1707 cm⁻¹ |
| CHO | 2735 cm⁻¹ |
| Aromatiques | 1600 - 1580 - 1570 - 1470 cm⁻¹ |

### Stade B : [[4-bromo 3-(trifluorométhyl) hydroxyméthyl] phosphonate de diéthyle

On introduit à 60°C environ, 12,65 g de 4-bromo 3-trifluorométhyl benzaldéhyde obtenu au stade A, dans un mélange renfermant 6,5 cm³ de diéthylphosphite et 0,5 cm³ de triéthylamine. On agite 1 heure à 70°C et refroidit à 40°C, on ajoute alors 100 cm³ de toluène. On lave avec une solution aqueuse d'acide chlorhydrique 1N, puis à l'eau. On sèche et évapore à sec. On obtient 17,4 g de produit. F = 88°C.

### Stade C : [[4-bromo 3-(trifluorométhyl) phényl] fluorométhyl] phosphonate de diéthyle

On ajoute à 0°C, 2,5 cm³ de trifluorure de diéthylamino sulfure dans un mélange renfermant 7,82 g de produit préparé au stade précédent en solution dans 50 cm³ de chlorure de méthylène. On maintient le mélange réactionnel à 20 ≃ 25°C pendant 1 heure. On le verse sur une solution aqueuse saturée en bicarbonate de sodium. On décante, extrait au chlorure de méthylène, sèche et amène à sec. On chromatographie le produit obtenu en éluant avec le mélange chlorure de méthylène-tétrahydrofuranne (98-2). On obtient 5,7 g du produit recherché. rf = 0,2.

### PREPARATION 3 : [[2-(3,4-dichlorophényl) 4-thiazolyl] fluoro méthyl] phosphonate de diéthyle

### STADE A : [[2-(3,4-dichlorophényl) 4-thiazolyl] hydroxyméthyl]] phosphonate de diéthyle

On chauffe à 60°C, un mélange de 3,9 cm³ de diéthylphosphite et 0,3 cm³ de triéthylamine. On introduit 12,65 g de produit 2-[2-(3,4-dichlorophényl 4-formyl thiazole obtenu selon EP 402246. On chauffe à 160°C, et laisse la température revenir à 110°C. On chauffe pendant 4 heures à 110°C. On laisse la température revenir à 20 ≃ 25°C. On verse le produit obtenu dans 1 litre de chlorure de méthylène. On verse le milieu réactionnel dans 100 cm³ d'une solution normale d'acide chlorhydrique. On décante la phase chlorométhylénique, sèche et amène à sec. On lave le produit obtenu à l'éther isopropylique et le sèche sous pression réduite. On obtient 5,9 g de produit recherché. F = 155°C.

### STADE B : [2-[2-(3,4-dichlorophényl) 4-thiazolyl] fluorométhyl] phosphonate de diéthyle

En opérant comme à la préparation précédente, à partir du produit obtenu au stade A, on obtient 2,4 g du produit recherché.

rf = 0,25 éluant : chlorure de méthylène-éther isopropylique (30-10).

### PREPARATION 4: [[2-[4-(trifluorométhoxy)] 4-thiazolyl] fluorométhyl] phosphonate de diéthyle

### Stade A : 2-[4-(trifluorométhoxy) 4-thiazolyl] hydroxy méthyl] phosphonate de diéthyle

On ajoute à 70°C, 2,74 g de 2-[2-[4-(trifluorométhoxy) phényl] 4-formyl thiazole obtenu selon EP 402246, dans une solution renfermant 1,3 cm³ de diéthylphosphite et 0,1 cm³ de triéthylamine. On laisse refroidir à 40°C et ajoute 15 cm³ de toluène. On lave avec une solution normale d'acide chlorhydrique puis à l'eau. On extrait les phases aqueuses avec du chlorure de méthylène. On réunit les phases organiques et les sèche. On filtre, rince et élimine le solvant. On obtient 2,87 g de produit recherché (isolé du pentane).

rf = 0,08 (chlorure de méthylène-tétrahydrofuranne (9-1).

### Stade B : [[2-[4-(trifluorométhoxy) 4-thiazolyl] fluorométhyl] phosphonate de diéthyle

On ajoute à -5°C, 2 cm³ de trifluorure de diéthylamino sulfure à 95 %, dans une solution renfermant 6,1 g de [[2-[4-(trifluorométhoxy) 4-thiazolyl] hydroxy méthyl] phosphonate de diéthyle. On verse le mélange réactionnel dans une solution aqueuse saturée de bicarbonate de sodium. On extrait au chlorure de méthylène. On réunit les phases organiques, les sèche et évapore le solvant. On obtient 6,42 g d'un produit que l'on chromatographie sur silice en éluant avec le mélange chlorure de méthylène-éther isopropylique (9-1). On obtient ainsi 2,3 g de produit recherché.

### PREPARATION 5: [fluoro [4-phényl 2-thiazolyl] méthyl] phosphonate de diéthyle.

### Stade A : Ester diéthylique de l'acide [(aminocarbonyl) fluorométhyl] phosphonique

On introduit à 5/10°C, sous arrivée d'azote et sous agitation, 700 cm³ d'ammoniaque 22°Be dans 664 g de (diéthoxyphosphinyl) fluoro acétate de méthyle. On maintient le mélange réactionnel sous agitation pendant 3 heures. On distille l'excès d'ammoniaque sous pression réduite. On sature le milieu réactionnel en chlorure de sodium. On extrait au chlorure de méthylène. On lave avec une solution saturée en chlorure de sodium. On sèche la phase chlorométhylénique. On filtre et amène à sec sous pression réduite. On obtient 646 g de produit recherché brut que l'on dissout dans 1 litre de chlorure de méthylène. On distille sous pression réduite à volume constant, en remplaçant le chlorure de méthylène par l'éther isopropylique. On essore, lave et sèche. On obtient 551,4 g de produit recherché.

### Stade B : [(2-amino 1-fluoro 2-thioxo) éthyl] phosphonate de diéthyle

On agite pendant 5 heures à la température ambiante une suspension de 57,9 g de [2-amino 1-fluoro 2-oxo] éthyl] phosphonate de diéthyle, obtenu au stade A, 580 cm³ de tétrahydrofuranne et 16 g de pentasulfure de phosphore. On filtre et évapore le solvant. On obtient 84,55 g de produit que l'on chromatographie (éluant : acétone-cyclohexane (1-1). On obtient 26,6 g du produit recherché.

### Stade C : [fluoro [4-phényl 2-thiazolyl] méthyl] phosphonate

### de diéthyle

On porte au reflux pendant 1 h 30, un mélange de 2,3 g de produit préparé au stade B, 30 cm³ de méthanol et 2 g d'alphabromo acétophénone. On laisse refroidir et verse dans un mélange eau-acétate d'éthyle (100 cm³ - 50 cm³). On neutralise le milieu réactionnel. On décante et extrait à l'acétate d'éthyle. On lave à l'eau, sèche et évapore. On obtient 3,18 g de produit que l'on chromatographie sur silice : éluant chlorure de méthylène-tétrahydrofuranne (98-2). On obtient ainsi 2,47 g de produit recherché.

### PREPARATION 6: [fluoro [4-(1-méthyléthyl) 2-thiazolyl] méthyl] phosphonate de diéthyle

En opérant comme à la préparation 5, stade C, à partir de 1-bromo 3-méthyl 2-butanone, on obtient 4,13 g du produit recherché. rf = 0,23 (chlorure de méthylène-tétrahydrofuranne (95-5)).

### PREPARATION 7: [[4-(3,4-dichlorophényl) 2-thiazolyl] fluorométhyl] phosphonate de diéthyle

### Stade A : 3',4'-dichloro bromoacétophénone

On agite 3 heures au reflux, 55,85 g de bromure cuivrique avec 23,75 g de 3',4'-dichloroacétophénone dans 125 cm³ de chloroforme et 125 cm³ d'acétate d'éthyle contenant des traces d'acide bromhydrique. On filtre, traite le filtrat avec 30 g de charbon actif, filtre à nouveau et concentre sous pression réduite, reprend avec 40 cm³ d'éther éthylique. On obtient 21,3 g du produit recherché. F = 52°C, utilisé tel quel pour le stade suivant.

### Stade B : [[4-(3,4-dichlorophényl) 2-thiazolyl] fluorométhyl] phosphonate de diéthyle

On porte au reflux pendant 2 heures 45 minutes, un mélange de 2,3 g du produit préparé à la préparation 5, stade A ou [(2-amino 1-fluoro 2-thioxo) éthyl] phosphonate de diéthyle, 30 cm³ de méthanol et 3,4 g de dichloro alpha-bromo acétophénone, obtenu au stade A ci-dessus. On agite vigoureusement et neutralise. On décante, extrait à l'acétate d'éthyle, lave à l'eau, sèche et évapore le solvant. On obtient 3,64 g de produit brut que l'on chromatographie en éluant avec le mélange chlorure de méthylène-tétrahydrofuranne (98-2). On obtient 2,8 g du produit recherché. rf = 0,3.

### PREPARATION 8: [fluoro [2-(trifluorométhyl) 4-thiazolyl] méthyl] phosphonate de diéthyle.

On opère comme indiqué à la préparation 2 stades B et C en utilisant au départ l'aldéhyde approprié. On obtient le produit attendu Rf = 0,2 (chlorure de méthylène-tétrahydrofuranne 98-2).

### PREPARATION 9: [4-[[1,1'-biphényl] 4-yl] 2-thiazolyl] fluorométhyl] phosphonate de diéthyle.

On porte au reflux pendant 7 heures un mélange de 3,4 g de produit préparé au stade B de la préparation 5, 45 cm³ de méthanol et 4,1 g de 1-[(1,1'-biphényl) 4-yl] 2-bromoéthanone. On laisse refroidir et verse dans un mélange eau-acétate d'éthyle (200 cm³-100 cm³). On neutralise le milieu réactionnel. On décante et extrait à l'acétate d'éthyle. On lave à l'eau, sèche et évapore. On obtient 6,1 g de produit brut que l'on recristallise dans du pentane et recueille 5 g de produit attendu. F = 91,2°C.

### PREPARATION 10: [[4-(4-chlorophényl) 2-thiazolyl] fluorométhyl] phosphonate de diéthyle.

On opère comme indiqué au stade C de la préparation 5 en utilisant au départ 3,44 g du phosphonate préparé au stade B et 3,5 g de 2-bromo 4'-chloroacétophénone. On obtient 2,83 g de produit attendu. Rf = 0,17 (chlorure de méthylène-tétrahydrofuranne 98-2).

### PREPARATION 11: [[4-(4-bromophényl) 2-thiazolyl] fluorométhyl] phosphonate de diéthyle.

On opère comme indiqué au stade C de la préparation 5 en utilisant au départ 3,44 g du phosphonate préparé au stade B 4,17 g de 2-bromo 4'-bromoacétophénone. On obtient 4,6 g de produit attendu. Rf = 0,22 (chlorure de méthylène-tétrahydrofuranne 98-2).

### PREPARATION 12: [fluoro [4-(trifluorométhyl 2-thiazolyl] méthyl] phosphonate de diéthyle.

On mélange 6,9 g du phosphonate obtenu au stade B de la préparation 5, et 5,8 g de 1-bromo 3,3,3-trifluoroacétophénone. On dilue avec 20 cm³ de chlorure de méthylène, lave à l'eau, sèche, évapore le solvant, chromatographie le résidu sur silice (éluant : chlorure de méthylène-tétrahydrofuranne 98-2) et obtient 2,2 g de produit attendu. Rf = 0,05 (hexane-acétate d'éthyle 7-3).

### PREPARATION 13 : [2'-éthyl (2,4'-bithiazole) 4-yl] fluorométhyl phosphonate de diéthyle.

On opère comme indiqué à la préparation 2 stades B et C en utilisant au départ l'aldéhyde approprié. On obtient le produit attendu. Rf = 0,05 (hexane-acétate d'éthyle 7-3).

### PREPARATION 14 : [fluoro [2-(2-pyridinyl) 4-thiazolyl) méthyl] phosphonate de diéthyle.

On opère comme indiqué à la préparation 2 stades B et C en utilisant au départ l'aldéhyde approprié. On obtient le produit attendu. Rf = 0,47 (chlorure de méthylène-tétrahydrofuranne 85-15).

### PREPARATION 15 : [fluoro [3-[4-(trifluorométhoxy) phényl] 1,2,4-oxadiazol-5-yl] méthyl] phosphonate de diéthyle.

### Stade A : N-hydroxy 4-(trifluorométhoxy) benzène carboximidamine.

On ajoute en 5 minutes 3,8 cm³ de triéthylamine à 2,05 g de chlorhydrate d'hydroxylamine dans 30 cm³ de méthanol et agite 30 minutes à température ambiante. On ajoute alors 5 g de 4-trifluorométhoxy benzonitrile dans 10 cm³ de méthanol et chauffe 50 minutes au reflux. On refroidit, évapore le solvant, reprend le résidu au chlorure de méthylène, lave à l'eau, sèche et élimine le solvant sous pression réduite. On obtient 5 g de produit attendu. F = 111°C.

### Stade B : [fluoro [3-[4-(trifluorométhoxy) phényl] 1,2,4-oxadiazol-5-yl] méthyl] phosphonate de diéthyle.

On mélange à température ambiante 4,93 g d'oxime préparée au stade A et 4,8 g d'acide (diéthoxy phosphoryl) fluoro acétique dans 90 cm³ de chlorure de méthylène. On ajoute goutte à goutte 5,8 g de dicyclohexylcarbodiimide et 140 mg de diméthylaminopyridine dans 20 cm³ de chlorure de méthylène. On filtre l'urée formée, évapore le solvant et obtient 11,45 g de produit auquel on ajoute 30 cm³ de toluène et chauffe 1 heure au reflux. On refroidit, évapore le solvant, chromatographie le résidu sur silice (éluant : hexane-tétrahydrofuranne 7-3) et obtient 6,45 g de phosphonate attendu.

### PREPARATION 16: [fluoro [3-(trifluorométhyl) phényl] méthyl] phosphonate de diéthyle.

On opère comme à la préparation 2 stades B et C à partir du 3-trifluorométhyl benzaldéhyde. On obtient le produit attendu. Rf = 0,17 (chlorure de méthylène-tétrahydrofuranne 98-2).

### PREPARATION 17: [fluoro [4-(4-nitrophényl) 2-thiazolyl] méthyl] phosphonate de diéthyle.

On opère comme à la préparation 9 à partir de 5,5 g de [(2-amino 1-fluoro 2-thioxo) éthyl] phosphonate de diéthyle et 4,93 g de 2-bromo 3'-nitro acétophénone. On obtient 9,46 g de produit brut que l'on chromatographie sur silice (éluant : chlorure de méthylène-éther isopropylique 85-15) et récupère 4,22 g de produit attendu. F = 100°C.

### PREPARATION 18: [fluoro [4-(fluorophényl) 2-thiazolyl] méthyl] phosphonate de diéthyle.

On opère comme à la préparation 9 à partir de 36,6 g de [(2-amino 1-fluoro 2-thioxo) éthyl] phosphonate de diéthyle et 31,8 g de 2-bromo 4'-fluoro acétophénone. On obtient 56,8 g de produit brut que l'on chromatographie sur silice (éluant : chlorure de méthylène-tétrahydrofuranne 98-2) et récupère 28,58 g de produit attendu. F = 100°C.

### PREPARATION 19: [[5-bromo 4-(4-fluorophényl) 2-thiazolyl] fluorométhyl phosphonate de diéthyle.

### Stade A : [(2-amino 1-fluoro 2-thioxo) éthyl] phosphonate de diéthyle.

On ajoute 16 g de pentasulfure de phosphore à 57,9 g de [(2-amino 1-fluoro 2-oxo) éthyl] phosphonate de diéthyle dans 500 cm³ de tétrahydrofuranne puis agite 5 heures à température ambiante. On filtre, évapore le solvant, chromatographie sur silice (éluant : chloroforme-acétone-cyclohexane 1-1-1) et récupère 26,6 g de produit attendu. F = 52,6°C.

### Stade B : [4-(4-fluorophényl) 2-thiazolyl] fluorométhylphosphonate de diéthyle.

On chauffe 30 minutes au reflux 33,6 g de phosphonate obtenu au stade A, 500 cm³ d'éthanol et 31,8 g de 2-bromo 4-fluoro acétophénone. On refroidit à température ambiante, verse sur 500 cm³ d'eau et extrait à l'acétate d'éthyle. On évapore le solvant, chromatographie le résidu sur silice (éluant : chlorure de méthylène-tétrahydrofuranne 98-2) et récupère 28,58 g de produit attendu.

### Stade C : [[5-bromo 4-(4-fluorophényl) 2-thiazolyl] fluorométhyl phosphonate de diéthyle.

On chauffe 1 heure au reflux 21,7 g de phosphonate obtenu au stade B, 250 cm³ d'acétonitrile et 14,5 g de N-bromo succinimide. On refroidit, évapore le solvant et récupère après chromatographie sur silice (éluant : chlorure de méthylène-tétrahydrofuranne 98-2), 24 g de produit attendu.

### PREPARATION 20: [3-(trifluorométhyl) phénylchlorométhyl] phosphonate de diéthyle.

On opère comme à la préparation 2 stade B à partir du 3-trifluorométhyl benzaldéhyde pour obtenir le [3-(trifluorométhyl) hydroxyméthyl] phosphonate de diéthyle. A 6,26 g de ce phosphonate dans 60 cm³ de chlorure de méthylène, on ajoute 1,6 cm³ de pyridine puis en 20 minutes 1,6 cm³ de chlorure de thionyle et agite 1 heure à température ambiante. On chauffe à 40°C pendant 28 heures, refroidit à 20°/25°C, lave avec une solution aqueuse de bicarbonate de sodium puis à l'eau, sèche et évapore le solvant. On récupère 6,4 g de produit brut que l'on purifie par chromatographie sur silice (éluant : hexaneacétate d'éthyle 5-5). On obtient 5,15 g de produit attendu.

### PREPARATION 21: [3-(trifluorométhyl) phénylbromométhyl] phosphonate de diéthyle.

On refroidit à 0°/+5°C, 6,26 g de [3-(trifluorométhyl) hydroxyméthyl] phosphonate de diéthyle dans 60 cm³ de chlorure de méthylène, ajoute en 10 minutes 9,95 g de tétrabromocarbone, laisse remonter la température à +7°/+10°C, ajoute 7,9 g de triphénylphosphine, laisse revenir à température ambiante et agite pendant 48 heures. On filtre, verse le filtrat dans une solution aqueuse saturée de bicarbonate de sodium, agite 20 minutes, et extrait au chlorure de méthylène. On lave la phase organique à l'eau, la sèche, évapore le solvant et récupère 18 g de produit brut. On reprend dans un mélange hexane-acétate d'éthyle 5-5, filtre, évapore de nouveau le solvant, chromatographie le résidu sur silice (éluant : hexane-acétate d'éthyle 5-5) et recueille 5,55 g de produit attendu.

### EXEMPLES DE COMPOSITIONS INSECTICIDES

### EXEMPLE 31 : Préparation d'un concentré soluble

On effectue un mélange homogène de :

| | |
|---|---|
| Produit de l'exemple 1 (isomère Z,E) | 0,25 g |
| Tween 80^{®} | 0,25 g |
| Topanol A^{®} | 0,1 g |
| Eau | 98,4 g |

### EXEMPLE 32 : Préparation d'un concentré émulsifiable

On mélange intimement :

| | |
|---|---|
| Produit de l'exemple 7 (isomère Z,E) | 0,015 g |
| Topanol A^{®} | 0,1 g |
| Tween 80^{®} | 3,5 g |
| Xylène | 95,885 g |

### Etude de l'activité des composés de l'invention

### a) Etude de l'activité sur Aphis craccivora

Des plants de fèves sont traités par trempage des feuilles dans une solution hydroacétonique de matière active (50 % acétone, 50 % eau) puis séchées sous hotte aspirante.

Les feuilles sont ensuite infestées : 20 femelles d'Aphis craccivora adultes par feuille et maintenues à 22°C sous plafond lumineux.

Les contrôles de mortalité sont effectués au bout de 48 heures. **Résultats :** Dès la dose de 10 ppm, les produits des exemples 2, 3, 4, 7, 8, 10, 11, 12, 14, 16, 17, 18, 20, 21, 22, 24, 25, 26 et 30 présentent une efficacité ≥ 75%.

### b) Etude de l'effet sur larves de Spodoptera littoralis par contact et ingestion.

On utilise des larves du stade L3 de Spodoptera littoralis. On opère à 22°C dans des conditions d'humidité relative de 50 %. On utilise des boîtes de PETRI renfermant un rond de papier filtre humidifié, dans chaque boîte on place deux feuilles de haricot traitées par une solution hydroacétonique (50-50) renfermant le produit à tester.

On fait le comptage des larves mortes au bout de 7 jours. **Résultats :** Dès la dose de 10 ppm, les produits des exemples 2, 4, 7, 8, 9, 10, 11, 15, 16, 17, 18, 20, 25, 26 et 30 présentent une efficacité ≥ 75%.

### c) Etude de l'effet sur Phaedon cochleariae.

On opère à 22°C dans des conditions d'humidité relative de 50 %. On utilise des boîtes de PETRI renfermant un rond de papier filtre humidifié et deux disques de feuille de chou chinois traités par une solution hydroacétonique (50-50) renfermant le produit à tester.

On fait le comptage des insectes morts au bout d'une semaine. **Résultats** : Dès la dose de 10 ppm, les produits des exemples 10, 18, 25 et 26 présentent une efficacité ≥ 75%.

### d) Etude de l'effet sur Tetranychus urticae

On utilise des plants de haricot comportant 2 feuilles infestées de 30 femelles de Tetranychus urticae par feuille et mis sous bonette aérée sous plafond lumineux en lumière constante. Les plants sont traités au pistolet Fisher : 4 ml de solution toxique par plant d'un mélange à volume égal d'eau et d'acétone. On laisse sécher pendant une 1/2 heure puis on procède à l'infestation. Les contrôles de mortalité sont effectués au bout de 3 jours. **Résultats** : Dès la dose de 10 ppm, les produits des exemples 1, 2, 3, 4, 6, 8, 10, 11, 12, 13, 15, 16, 18, 20, 22, 25, 26 et 30 présentent une efficacité ≥ 75%.

## Revendications

1. Les composés de formule (I) : 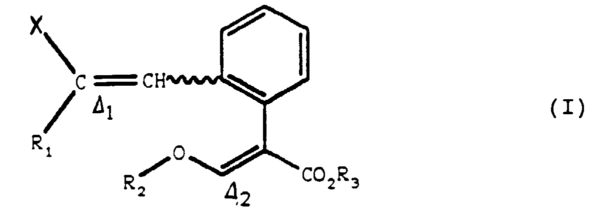 dans laquelle :
- X représente un atome d'halogène,
- R₂ et R₃ identiques ou différents l'un de l'autre représentent un radical alkyle linéaire, ramifié ou cyclique renfermant jusqu'à 8 atomes de carbone éventuellement substitué par un ou plusieurs atomes d'halogène, et
- ou R₁ représente un radical alkyle, alkényle ou alkynyle linéaire, ramifié ou cyclique, renfermant jusqu'à 12 atomes de carbone, éventuellement substitué par un ou plusieurs atomes d'halogène,
- ou R₁ représente un radical aryle ou hétéroaryle à 5 ou 6 chaînons, éventuellement substitués par un ou plusieurs substituants choisis dans le groupe constitué par les atomes d'halogène, par les radicaux alkyle, alkényle ou alkynyle linéaires, ramifiés ou cycliques ayant jusqu'à 8 atomes de carbone, eux-mêmes éventuellement substitués par un ou plusieurs atomes d'halogène, par les radicaux NO₂, NH₂, CN, OR', SR", SOR", SO₂R" et NR‴₂ (R', R" et R‴ représentant des radicaux alkyle, alkényle ou alkynyle linéaires, ramifiés ou cycliques, renfermant jusqu'à 12 atomes de carbone éventuellement substitués par un ou plusieurs atomes d'halogène) et par les radicaux Ar, OAr₁ et SAr₂, dans lesquels les radicaux Ar, Ar₁ et Ar₂ représentent des radicaux aryles ou hétéroaryles renfermant jusqu'à 14 atomes de carbone éventuellement substitués par un ou plusieurs substituants choisis dans le groupe constitué par les atomes d'halogène, par les radicaux alkyle, alkényle ou alkynyle linéaires, ramifiés ou cycliques ayant jusqu'à 8 atomes de carbone, eux-mêmes éventuellement substitués par un ou plusieurs atomes d'halogène, par les radicaux NO₂, NH₂, CN, OR', SR", SOR", SO₂R" et NR‴₂ (R', R" et R‴ représentant des radicaux alkyle, alkényle ou alkynyle linéaires, ramifiés ou cycliques, renfermant jusqu'à 12 atomes de carbone éventuellement substitués par un ou plusieurs atomes d'halogène) sous toutes les formes stéréoisomères possibles ainsi que leurs mélanges.

2. Les composés de formule (I) tels que définis à la revendication 1 dans lesquels la double liaison Δ₂ est de géométrie E.

3. Les composés de formule (I) tels que définis à la revendication 1 ou 2 dans lesquels R₂ représente un radical méthyle.

4. Les composés de formule (I) tels que définis à l'une quelconque des revendications 1 à 3 dans lesquels R₃ représente un radical méthyle.

5. Les composés de formule (I) tels que définis à l'une quelconque des revendications 1 à 4 dans lesquels X représente un atome de fluor.

6. Les composés de formule (I) tels que définis à l'une quelconque des revendications 1 à 5 dans lesquels R₁ représente un radical phényle éventuellement substitué par un ou plusieurs radicaux choisis dans le groupe constitué par les atomes d'halogène et les radicaux CF₃.

7. Les composés de formule (I) tels que définis à l'une quelconque des revendications 1 à 5 dans lesquels R₁ représente un radical thiazolyle éventuellement substitué par un ou plusieurs des radicaux indiqués à la revendication 1.

8. Les composé de formule (I) tels que définis à la revendication 7 dans lesquels R₁ est un radical thiazolyle substitué par un ou plusieurs radicaux choisis dans le groupe constitué par les atomes d'halogène et le radical phényle éventuellement substitué par un ou plusieurs radicaux choisis dans le groupe constitué par les atomes d'halogène et les radicaux CF₃ et OCF₃.

9. Les composés de formule (I) tels que définis à l'une quelconque des revendications 1 à 5 dans lesquels R₁ représente un radical oxadiazolyle éventuellement substitué par un ou plusieurs des radicaux indiqués à la revendication 1.

10. Les composés de formule (I) tels que définis à la revendication 9 dans lesquels R₁ est un radical oxadiazolyle substitué un radical phényle éventuellement substitué par un ou plusieurs radicaux choisis dans le groupe constitué par les atomes d'halogène et les radicaux CF₃ et OCF₃.

11. Les composés de formule (I) tels que définis à la revendication 1 dont les noms suivent :
- (E,E) 2-[2-[4-(3,4-dichlorophényl) 2-thiazolyl] 2-fluoro éthényl] alpha-(méthoxyméthylène) benzène acétate de méthyle,
- (Z,E) 2-[2-fluoro 2-(4-phényl 2-thiazolyl) éthényl] alpha(méthoxyméthylène) benzène acétate de méthyle,
- (E,E) 2-[2-[4-bromo 3-(trifluorométhyl) phényl] 2-fluoro éthényl] alpha-(méthoxyméthylène) benzène acétate de méthyle,
- (E,Z) 2-[2-[4-bromo 3-(trifluorométhyl) phényl] 2-fluoroéthényl] alpha-(méthoxyméthylène) benzène acétate de méthyle,
- (Z,E) 2-[2-[4-(3,4-dichlorophényl) 2-thiazolyl] 2-fluoro éthényl] alpha-(méthoxyméthylène) benzène acétate de méthyle.
- (Z,E) 2-[2-fluoro 2-[4-thiazolyl 2-(4-trifluoro méthoxy) phényl] éthényl] alpha-(méthoxyméthylène) benzène acétate de méthyle,
- (Z,E) 2-[2-[4-(4-fluorophényl) 2-thiazolyl] 2-fluoroéthényl] α-(méthoxyméthylène) benzèneacétate de méthyle,
- (Z,E) 2-[2-[5-bromo 4-(3,4-dichlorophényl) 2-thiazolyl] 2-fluoroéthényl] α-(méthoxyméthylène) benzèneacétate de méthyle,
- (Z,E) 2-[2-[5-bromo 4-(4-fluorophényl) 2-thiazolyl] 2-fluoroéthényl] α-(méthoxyméthylène) benzène acétate de méthyle,
- (Z,E) 2-[2-fluoro 2-[3-[4-(trifluorométhoxy) phényl] 1,2,4-oxadiazol-5-yl] éthényl] α-(méthoxyméthylène) benzène acétate de méthyle,
- Z,E) 2-[2-fluoro 2-[3-phényl 1,2,4-oxadiazol-5-yl] éthényl] α-(méthoxyméthylène) benzène acétate de méthyle.

12. Procédé de préparation des composés de formule (I) tels que définis à l'une quelconque des revendications 1 à 9 caractérisé en ce que l'on soumet un composé de formule (II) : 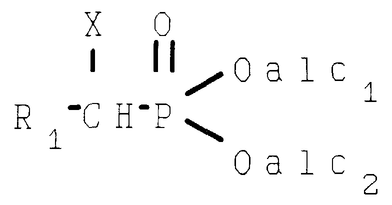 dans laquelle R₁ et X conservent leur signification précédente et alc₁ et alc₂ représentent un radical alkyle renfermant jusqu'à 8 atomes de carbone, à l'action d'un composé de formule (III) : 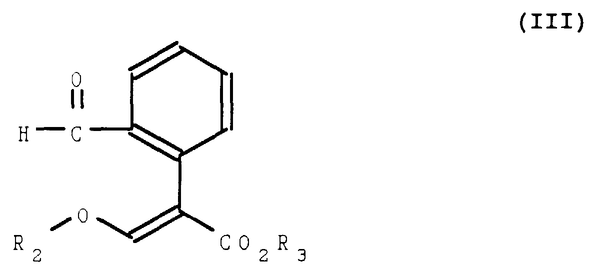 dans laquelle R₂ et R₃ conservent leur signification précédente, en présence d'un agent basique, pour obtenir le composé de formule (I) correspondant, que l'on sépare si désiré en chacun de ses isomères et le cas échéant transforme l'un des isomères en l'autre isomère.

13. Procédé de préparation selon la revendication 12, caractérisé en ce que l'isomérisation des doubles liaisons fluorées est réalisée au moyen d'une quantité catalytique de brome en présence de radiations lumineuses.

14. Les compositions pesticides renfermant comme principe actif au moins un composé de formule (I) défini à l'une quelconque des revendications 1 à 10.

15. Les compositions pesticides renfermant comme principe actif au moins un composé défini à la revendication 11.

16. Les compositions insecticides renfermant comme principe actif au moins un composé défini à l'une quelconque des revendications 1 à 11.

17. Les compositions acaricides renfermant comme principe actif au moins un composé défini à l'une quelconque des revendications 1 à 11.

18. Les compositions nématicides renfermant comme principe actif au moins un composé défini à l'une quelconque des revendications 1 à 11.

## Patentansprüche

1. Verbindungen der Formel (I) 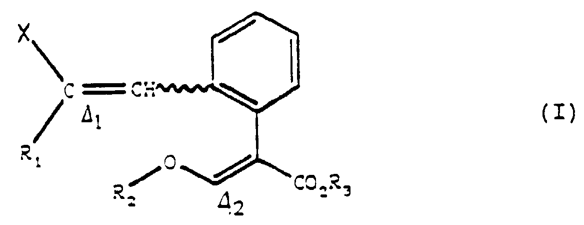 worin:
X ein Halogenatom bedeutet,
R₂ und R₃, die identisch oder voneinander verschieden sind, bedeuten einen linearen, verzweigten oder cyclischen Alkylrest mit bis zu 8 Kohlenstoffatomen, gegebenenfalls substituiert durch ein oder mehrere Halogenatom(e), und entweder R₁ bedeutet einen linearen, verzweigten oder cyclischen Alkyl-, Alkenyl- oder Alkinylrest mit bis zu 12 Kohlenstoffatomen, gegebenenfalls substituiert durch ein oder mehrere Halogenatom(e),
oder R₁ bedeutet einen Aryl- oder Heteroarylrest mit 5 oder 6 Kettengliedern, gegebenenfalls substituiert durch einen oder mehrere Substituenten, ausgewählt aus der Gruppe bestehend aus den Halogenatomen, aus den linearen, verzweigten oder cyclischen Alkyl-, Alkenyl- oder Alkinylresten mit bis zu 8 Kohlenstoffatomen, welche ihrerseits gegebenenfalls substituiert sind durch ein oder mehrere Halogenatom(e), durch die Reste NO₂, NH₂, CN, OR', SR", SOR", SO₂R" und NR"'₂ (wobei R', R" und R"' lineare, verzweigte oder cyclische Alkyl-, Alkenyl- oder Alkinylreste mit bis zu 12 Kohlenstoffatomen bedeuten, gegebenenfalls substituiert durch ein oder mehrere Halogenatom(e)) und durch die Reste Ar, OAr₁ und SAr₂, worin die Reste Ar, Ar₁ und Ar₂ Aryl- oder Heteroarylreste mit bis zu 14 Kohlenstoffatomen bedeuten, gegebenenfalls substituiert durch einen oder mehrere Substituenten, ausgewählt aus der Gruppe bestehend aus den Halogenatomen, aus den linearen, verzweigten oder cyclischen Alkyl-, Alkenyl- oder Alkinylresten bis bis zu 8 Kohlenstoffatomen, welche ihrerseits gegebenenfalls substituiert sind durch ein
oder mehrere Halogenatom(e), durch die Reste NO₂, NH₂, CN, OR', SR", SOR", SO₂R" und NR"'₂ (R', R" und R"' bedeuten lineare, verzweigte oder cyclische Alkyl-, Alkenyl- oder Alkinylreste mit bis zu 12 Kohlenstoffatomen, gegebenenfalls substituiert durch ein oder mehrere Halogenatom(e)) unter allen möglichen stereoisomeren Formen sowie ihre Gemische.

2. Verbindungen der Formel (I), wie in Anspruch 1 definiert, worin die Doppelbindung Δ₂ die E-Geometrie hat.

3. Verbindungen der Formel (I), wie in Anspruch 1 oder 2 definiert, worin R₂ einen Methylrest bedeutet.

4. Verbindungen der Formel (I), wie in einem der Ansprüche 1 bis 3 definiert, worin R₃ einen Methylrest bedeutet.

5. Verbindungen der Formel (I), wie in einem der Ansprüche 1 bis 4 definiert, worin X ein Fluoratom bedeutet.

6. Verbindungen der Formel (I), wie in einem der Ansprüche 1 bis 5 definiert, worin R₁ einen Phenylrest bedeutet, gegebenenfalls substituiert durch einen oder mehrere Reste, ausgewählt aus der Gruppe bestehend aus den Halogenatomen und den Resten CF₃.

7. Verbindungen der Formel (I), wie in einem der Ansprüche 1 bis 5 definiert, worin R₁ einen Thiazolylrest bedeutet, gegebenenfalls substituiert durch einen oder mehrere Rest(e), die in Anspruch 1 angegeben sind.

8. Verbindungen der Formel (I), wie in Anspruch 7 definiert, worin R₁ einen Thiazolylrest bedeutet, substituiert durch einen oder mehrere Reste ausgewählt aus der Gruppe bestehend aus den Halogenatomen und dem Phenylrest gegebenenfalls substituiert durch einen oder mehrere Rest(e) ausgewählt aus der Gruppe bestehend aus den Halogenatomen und den Resten CF₃ und OCF₃.

9. Verbindungen der Formel (I), wie in einem der Ansprüche 1 bis 5 definiert, worin R₁ einen Oxadiazolylrest bedeutet, gegebenenfalls substituiert durch einen oder mehrere der in Anspruch 1 angegebenen Reste.

10. Verbindungen der Formel (I), wie in Anspruch 9 definiert, worin R₁ einen Oxadiazolylrest bedeutet, substituiert durch einen Phenylrest gegebenenfalls substituiert durch einen oder mehrere Rest(e) ausgewählt aus der Gruppe bestehend aus den Halogenatomen und den Resten CF₃ und OCF₃.

11. Verbindungen der Formel (I), wie in Anspruch 1 definiert, mit den folgenden Bezeichnungen:
(E,E)-Methyl-2-[2-[4-(3,4-dichlorophenyl)-2-thiazolyl]-2-fluoroethenyl] -alpha-(methoxymethylen)-benzol-acetat,
(Z,E)-Methyl-2-[2-fluoro-(4-phenyl-2-thiazolyl)-ethenyl]-alpha-(methoxymethylen)-benzol-acetat,
(E,E)-Methyl-2-[2-[4-bromo-3-(trifluoromethyl)-phenyl]-2-fluoroethenyl]-alpha-(methoxymethylen)-benzol-acetat,
(E,Z)-Methyl-2-[2-[4-bromo-3-(trifluoromethyl)-phenyl]-2-fluoroethenyl]-alpha-(methoxymethylen)-benzol-acetat,
(Z,E)-Methyl-2-[2-[4-(3,4-dichlorophenyl)-2-thiazolyl]-2-fluoroethenyl]-alpha-(methoxymethylen)-benzol-acetat,
(Z,E)-Methyl-2-[2-fluoro-2-[4-thiazolyl-2-(4-trifluoromethoxy)-phenyl]-ethenyl]-alpha-(methoxymethylen)-benzolacetat,
(Z,E)-Methyl-2-[2-[4-(4-fluorophenyl)-2-thiazolyl]-2-fluoroethenyl]-α-(methoxymethylen)-benzol-acetat,
(Z,E)-Methyl-2-[2-[5-bromo-4-(3,4-dichlorophenyl)-2-thiazolyl]-2-fluoroethenyl]-α-(methoxymethylen)-benzol-acetat,
(Z,E)-Methyl-2-[2-[5-bromo-4-(4-fluorophenyl)-2-thiazolyl]-2-fluoroethenyl]-α-(methoxymethylen)-benzol-acetat,
(Z,E)-Methyl-2-[2-fluoro-2-[3-[4-(trifluoromethoxy)-phenyl]-1,2,4-oxadiazol-5-yl]-ethenyl]-α-(methoxymethylen)-benzolacetat,
(Z,E)-Methyl-2-[2-fluoro-2-[3-phenyl-1,2,4-oxadiazol-5-yl]-ethenyl]-α-(methoxymethylen)-benzol-acetat.

12. Verfahren zur Herstellung der Verbindungen der Formel (I), wie in einem der Ansprüche 1 bis 9 definiert, dadurch gekennzeichnet, daß man eine Verbindung der Formel (II): 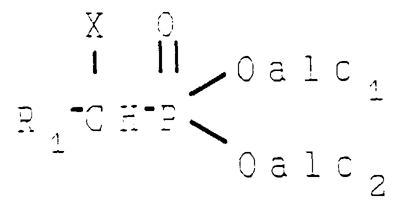 worin R₁ und X die vorstehende Bedeutung haben und alc₁ und alc₂ einen Alkylrest mit bis zu 8 Kohlenstoffatomen darstellen, der Einwirkung einer Verbindung der Formel (III): 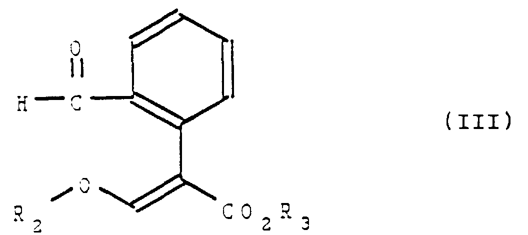 worin R₂ und R₃ die vorstehende Bedeutung haben, in Gegenwart eines basischen Mittels unterwirft, um die entsprechende Verbindung der Formel (I) zu erhalten, die man gewünschtenfalls in jede ihrer Isomeren auftrennt, und gegebenenfalls eines der Isomeren in das andere Isomere überführt.

13. Herstellungsverfahren gemäß Anspruch 12, dadurch gekennzeichnet, daß die Isomerisierung der fluorierten Doppelbindungen mittels einer katalytischen Brommenge in Gegenwart von Bestrahlungen durch Licht unterwirft.

14. Pestizide Zusammensetzungen, die als Wirkstoff mindestens eine Verbindung der Formel (I), wie in einem der Ansprüche 1 bis 10 definiert, enthalten.

15. Pestizide Zusammensetzungen, die als Wirkstoff mindestens eine Verbindung wie in Anspruch 11 definiert enthalten.

16. Insektizide Zusammensetzungen, die als Wirkstoff mindestens eine Verbindung wie in einem der Ansprüche 1 bis 11 definiert enthalten.

17. Akarizide Zusammensetzungen, die als Wirkstoff mindestens eine Verbindung wie in einem der Ansprüche 1 bis 11 definiert enthalten.

18. Nematizide Zusammensetzungen, die als Wirkstoff mindestens eine Verbindung wie in einem der Ansprüche 1 bis 11 definiert enthalten.

## Claims

1. The compounds of formula (I): 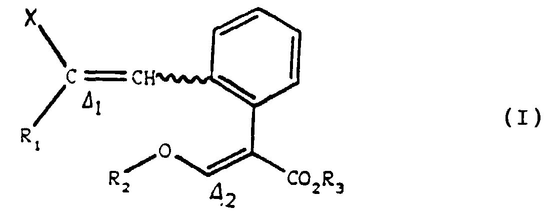 in which:
- X represents a halogen atom,
- R₂ and R₃, identical to or different from each other, represent a linear, branched or cyclic alkyl radical containing up to 8 carbon atoms optionally substituted by one or more halogen atoms, and
- either R₁ represents a linear, branched or cyclic alkyl, alkenyl or alkynyl radical containing up to 12 carbon atoms, optionally substituted by one or more halogen atoms,
- or R₁ represents an aryl or heteroaryl radical with 5 or 6 members, optionally substituted by one or more substituents chosen from the group constituted by halogen atoms, linear, branched or cyclic alkyl, alkenyl or alkynyl radicals having up to 8 carbon atoms, themselves optionally substituted by one or more halogen atoms, by the following radicals: NO₂, NH₂, CN, OR', SR", SOR", SO₂R" and NR"'₂ (R', R" and R"' representing linear, branched or cyclic alkyl, alkenyl or alkynyl radicals, containing up to 12 carbon atoms, optionally substituted by one or more halogen atoms) and by the Ar, OAr₁ and SAr₂ radicals, in which the Ar, Ar₁ and Ar₂ radicals represent aryl or heteroaryl radicals containing up to 14 carbon atoms optionally substituted by one or more substituents chosen from the group constituted by halogen atoms, by linear, branched or cyclic alkyl, alkenyl or alkynyl radicals having up to 8 carbon atoms, themselves optionally substituted by one or more halogen atoms, by the NO₂, NH₂, CN, OR', SR", SOR", SO₂R" and NR"'₂ radicals (R', R" and R"' representing linear, branched or cyclic alkyl, alkenyl or alkynyl radicals containing up to 12 carbon atoms, optionally substituted by one or more halogen atoms) in all the possible stereoisomeric forms as well as their mixtures.

2. The compounds of formula (I) as defined in claim 1 in which the Δ₂ double bond is of E geometry.

3. The compounds of formula (I) as defined in claim 1 or 2 in which R₂ represents a methyl radical.

4. The compounds of formula (I) as defined in any one of claims 1 to 3 in which R₃ represents a methyl radical.

5. The compounds of formula (I) as defined in any one of claims 1 to 4 in which X represents a fluorine atom.

6. The compounds of formula (I) as defined in any one of claims 1 to 5 in which R₁ represents a phenyl radical optionally substituted by one or more radicals from the group constituted by halogen atoms and the CF₃ radicals.

7. The compounds of formula (I) as defined in any one of claims 1 to 5 in which R₁ represents a thiazolyl radical optionally substituted by one or more of the radicals indicated in claim 1.

8. The compounds of formula (I) as defined in claim 7 in which R₁ is a thiazolyl radical substituted by one or more radicals chosen from the group constituted by halogen atoms and the phenyl radical optionally substituted by one or more radicals chosen from the group constituted by halogen atoms and CF₃ and OCF₃ radicals.

9. The compounds of formula (I) as defined in any one of claims 1 to 5 in which R₁ represents an oxadiazolyl radical optionally substituted by one or more of the radicals indicated in claim 1.

10. The compounds of formula (I) as defined in claim 9 in which R₁ is an oxadiazolyl radical substituted by a phenyl radical optionally substituted by one or more radicals chosen from the group constituted by halogen atoms and the CF₃ and OCF₃ radicals.

11. The compounds of formula (I) as defined in claim 1 the names of which follow:
- (E,E) methyl 2-[2-[4-(3,4-dichlorophenyl 2-thiazolyl] 2-fluoroethenyl] alpha-(methoxymethylene) benzene acetate,
- (Z,E) methyl 2-[2-fluoro 2-(4-phenyl 2-thiazolyl) ethenyl] alpha-(methoxymethylene) benzene acetate,
- (E,E) methyl 2-[2-[4-bromo 3-(trifluoromethyl) phenyl] 2-fluoroethenyl] alpha-(methoxymethylene) benzene acetate,
- (E,Z) methyl 2-[2-[4-bromo 3-(trifluoromethyl) phenyl] 2-fluoroethenyl] alpha-(methoxymethylene) benzene acetate,
- (Z,E) methyl 2-[2-[4-(3,4-dichlorophenyl) 2-thiazolyl] 2-fluoroethenyl] alpha-(methoxymethylene) benzene acetate,
- (Z,E) methyl 2-[2-fluoro 2-[4-thiazolyl 2-(4-trifluoro methoxy) phenyl] ethenyl] alpha-(methoxymethylene) benzene acetate,
- (Z,E) methyl 2-[2-[4-(4-fluorophenyl) 2-thiazolyl] 2-fluoroethenyl] alpha-(methoxymethylene) benzene acetate,
- (Z,E) methyl 2-[2-[5-bromo 4-(3,4-dichlorophenyl) 2-thiazolyl] 2-fluoroethenyl] alpha-(methoxymethylene) benzene acetate,
- (Z,E) methyl 2-[2-[5-bromo 4-(4-fluorophenyl) 2-thiazolyl] 2-fluoroethenyl] alpha-(methoxymethylene) benzene acetate
- (Z,E) methyl 2-[2-fluoro 2-[3-[4-(trifluoromethoxy) phenyl] 1,2,4-oxadiazol-5-yl] ethenyl] alpha-(methoxymethylene) benzene acetate,
- (Z,E) methyl 2-[2-fluoro 2-[3-phenyl 1,2,4-oxadiazol-5-yl] ethenyl] alpha-(methoxymethylene) benzene acetate.

12. Preparation process for the compounds of formula (I) as defined in any one of claims 1 to 9, characterized in that a compound of formula (II): 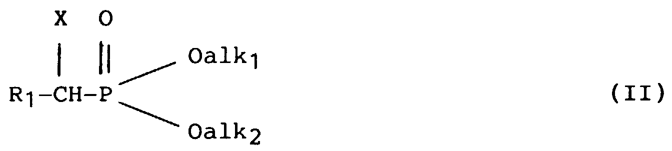 in which R₁ and X retain their previous meaning and alk₁ and alk₂ represent an alkyl radical containing up to 8 carbon atoms, is subjected to the action of a compound of formula (III): 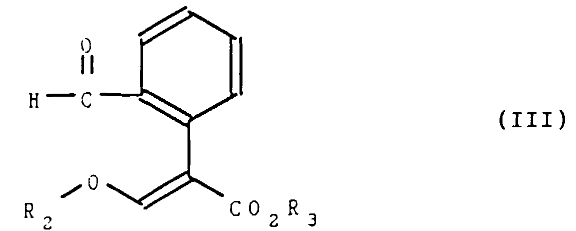 in which R₂ and R₃ retain their previous meaning, in the presence of a basic agent, in order to obtain the corresponding compound of formula (I), which, if desired, is separated into each of its isomers and if appropriate one of the isomers is converted into the other isomer.

13. Preparation process according to claim 12, characterized in that the isomerization of the fluorinated double bonds is carried out by means of a catalytic quantity of bromine in the presence of luminous radiation.

14. The pesticide compositions containing as active ingredient at least one compound of formula (I) defined in any one of claims 1 to 10.

15. The pesticide compositions containing as active ingredient at least one compound defined in claim 11.

16. The insecticide compositions containing as active ingredient at least one compound defined in any one of claims 1 to 11.

17. The acaricide compositions containing as active ingredient at least one compound defined in any one of claims 1 to 11.

18. The nematicide compositions containing as active ingredient at least one compound defined in any one of claims 1 to 11.
